# EUROPÄISCHE PATENTSCHRIFT

(11) **EP 3 535 003 B1**
(45) Veröffentlichungstag und Bekanntmachung des Hinweises auf die Patenterteilung: **04.08.2021**
(21) Anmeldenummer: 17788236.2
(22) Anmeldetag: 25.10.2017
(51) Int. Cl.: A61M 16/04

(54) **BIEGBARE TRACHEALBEATMUNGSVORRICHTUNG**
BENDABLE TRACHEAL VENTILATION APPARATUS
DISPOSITIF DE RESPIRATION ARTIFICIELLE TRACHÉALE SOUPLE

(30) Priorität: 02.11.2016 DE 102016120822
(43) Veröffentlichungstag der Anmeldung: 11.09.2019
(73) Patentinhaber: Tracoe Medical GmbH, 55268 Nieder-Olm (DE)
(72) Erfinder: SCHNELL, Ralf, 63500 Seligenstadt (DE); HAHN, Andreas, 55291 Saulheim (DE)
(74) Vertreter: WSL Patentanwälte Partnerschaft mbB
(86) Internationale Anmeldenummer: PCT/EP2017/077262
(87) Internationale Veröffentlichungsnummer: WO 2018/082982

(56) Entgegenhaltungen:
- WO-A1-2006/100424
- WO-A1-2010/062603
- WO-A1-2013/109569
- DE-A1-102006 035 887
- US-A- 4 852 564
- US-A- 4 987 895
- US-A1- 2002 043 266
- US-A1- 2010 163 050

## Beschreibung

Die vorliegende Erfindung betrifft eine Trachealbeatmungsvorrichtung, insbesondere eine Tracheostomiekanüle oder einen Endotrachealtubus, mit einem Kanülenrohr, welches einen proximalen Rohrabschnitt mit einer proximalen Öffnung und einen distalen Rohrabschnitt mit einer distalen Öffnung aufweist.

Derartige Trachealbeatmungsvorrichtungen werden in die Trachea eines Patienten eingeführt, um den Patienten mit Luft bzw. Atemgas zu versorgen. Das proximale Ende der Trachealbeatmungsvorrichtung ist außerhalb des Patienten mit einer Beatmungsvorrichtung verbindbar, während das distale Ende der Trachealbeatmungsvorrichtung in die Trachea einführbar ist. Das die zwei Öffnungen verbindende Kanülenrohr, dient der Hindurchleitung des Atemgases (Atemluft). Im Falle einer Tracheostomiekanüle wird die Kanüle durch eine künstlich erzeugte Öffnung in die Trachea eines Patienten eingeführt. Im Falle eines Endotrachealtubus wird diese Kanüle über den Mund und Rachenraum in die Trachea eingebracht. Die hier diskutierten Probleme treten bei beiden Typen von Trachealtuben auf, vornehmlich jedoch bei Tracheostomiekanülen. Zur Nutzung von Tracheostomiekanülen wird eine Öffnung (Stoma) vom Hals zur Trachea angelegt. In diese Öffnung wird die Tracheostomiekanüle eingesetzt, wobei die Kanüle in einem etwa 90° umfassenden Bogen von der Außenseite des Patientenhalses in die Trachea verläuft. Da die Anatomie von jedem Patienten unterschiedlich ist, kann es hier jedoch zu Passungsproblemen kommen, die beispielsweise dazu führen, dass das distale Kanülenende an der Tracheawand reibt.

Ein weiteres Problem besteht darin, dass eine in die Trachea eingeführte Trachealbeatmungsvorrichtung grundsätzlich den Schluckprozess eines Patienten erschwert, da sich zum Schlucken der Kehlkopf mit der Trachea etwas anhebt und senkt. Das Gewicht, die Form und die Größe der Trachealbeatmungsvorrichtung schränken jedoch die Beweglichkeit der Trachea und des Kehlkopfs ein. Ist die Trachealbeatmungsvorrichtung unnachgiebig bzw. aus einem harten Material hergestellt, besteht das Risiko, dass das distale Ende der Trachealbeatmungsvorrichtung beim Schlucken gegen die Tracheawand drückt. Dies kann zu Nekrosen und Blutungen führen. Um solche Effekte zu vermeiden gibt es sehr weiche Tracheostomiekanülen. Bei solchen weichen Trachealbeatmungsvorrichtungen ist das Einführen in die Trachea meist mühsam. Die Trachealbeatmungsvorrichtung kann abknicken bzw. verdrehen und die Kanülenspitze kann in einem ungünstigen Winkel auf die Trachealwand auftreffen und daran reiben. Hierdurch kann die Trachea beschädigt werden. Im ungünstigsten Fall kann es bei weichen Kanülen sogar vorkommen, dass die Kanüle beim Einführen nicht in Richtung Lunge sondern fälschlicherweise in Richtung Mund eingeführt wird.

Aus der US 2010/0163050 ist eine Tracheostomiekanüle bekannt, bei welcher ein proximaler und ein distaler Abschnitt der Kanüle unterschiedliche Härten aufweisen. Der distale Abschnitt soll dabei weicher und leichter beweglich sein. In einer Ausführungsform wird auch ein Übergangsbereich beschrieben, in welchem die Härte des Kunststoffs von dem härteren, proximalen Material allmählich in das weichere, distale Material übergeht. Dieser Übergangsbereich ebenso wie die abrupten Übergänge anderer Ausführungsformen zwischen hartem und weichem Material liegen proximal von dem Cuff.

Aus der WO 2013/109569 A1 ist ein Endotrachealtubus bekannt, welcher der Erfassung von Ödemen in den Luftwegen dient und zur Beseitigung von Engstellen einen radial faltbaren und dehnbaren Stentabschnitt aufweist. Dieser Abschnitt liegt ebenfalls proximal von einem zusätzlich vorgesehenen Cuff.

Die DE 10 2006 035 887 A1 beschreibt eine Trachealkanüle mit sogenannten Fensteröffnungen proximal von einem Cuff sowie einen dehnbaren Bereich, der wiederum proximal von den Fensteröffnungen angeordnet ist.

Die US 4,852,564 beschreibt einen Endotrachealtubus mit einem Cuff, der außerhalb des Körpers eines Patienten einen ziehharmonikaartig beweglichen Anschluss aufweist.

Die US 4,987,895 beschreibt eine ähnliche Vorrichtung als Trachealtubus, wobei der proximal zu einem Cuff liegende Bereich ziehharmonikaartig und damit gelenkig bewegbar ist.

Die WO 2006/100424 beschreibt eine Trachealkanüle, die durchgehend ein verstärktes Gerippe mit weicheren Teilen dazwischen aufweist. Ein distaler Abschnitt wird von einem durchgehenden Rohr jenseits eines Cuffs gebildet.

US 2002/0043266 A1 beschreibt einen Endotrachealtubus, dessen distales Ende durch einen Mechanismus gebogen werden kann, um das Einführen und die Platzierung des Tubus zu erleichtern. Der hierfür vorgesehene Gelenkabschnitt besteht vorzugsweise aus einer im Bereich des Cuffs angeordneten Spiralfeder.Vor diesem Hintergrund ist es eine Aufgabe der vorliegenden Erfindung, eine einfach handhabbare, leicht einführbare und einen Schluckprozess möglichst wenig erschwerende Trachealbeatmungsvorrichtung bereitzustellen, bei der das Risiko die Trachealwand zu verletzen reduziert wird.

Erfindungsgemäß wird die Aufgabe durch eine Trachealbeatmungsvorrichtung mit den Merkmalen des Anspruchs 1 gelöst. Der proximale Rohrabschnitt und der distale Rohrabschnitt sind dabei durch ein axial luftdurchlässiges Gelenk miteinander verbunden sind, welches mindestens eine begrenzte Bewegbarkeit und insbesondere Verschwenkbarkeit der Achse des distalen Rohrabschnittes bezüglich der Achse des proximalen Rohrabschnitts ermöglicht.

Durch das Gelenk sind der proximale Rohrabschnitt und der distale Rohrabschnitt relativ zueinander bewegbar, d. h. die Achsen der beiden Kanülenabschnitte sind verschwenkbar und unter unterschiedlichen relativen Winkeln einstellbar. Als Achse werden hier die zentralen Längsmittellinien der rohrförmigen Kanülenabschnitte bezeichnet, die auch bogenförmig gekrümmt sein können und die ohne Gelenk zwischen dem proximalen und dem distalen Abschnitt glatt und ohne Knick gegebenenfalls in einer leichten Krümmung ineinander übergehen würden, wobei das Gelenk eine relativ Verschwenkung bzw. einen leichten Knick das Achsenverlaufes mit einem vergleichsweise kleine Krümmungsradius ermöglichen soll, der weniger als die Hälfte, vorzugsweise weniger als ein Fünftel des sonstigen minimalen Krümmungsradius des Kanülenrohres beträgt. Der distale Rohrabschnitt kann somit gegenüber dem proximalen Rohrabschnitt in verschiedene Richtungen verschwenkt werden. Während des Einführens der Trachealbeatmungsvorrichtung in die Trachea und auch danach kann sich der proximale Rohrabschnitt zusammen mit dem Kehlkopf und der Trachea bewegen, ohne dass der distale Rohrabschnitt des Kanülenrohrs gegen die Wand der Trachea drückt oder an dieser reibt. Die gelenkige Verbindung des proximalen Rohrabschnittes mit dem distalen Rohrabschnitt erhöht die Beweglichkeit der Trachealbeatmungsvorrichtung und erleichtert das Schlucken.

Das Kanülenrohr weist außerdem auf seinem äußeren Umfang einen befüllbaren Cuff zur Abdichtung und weichen Fixierung des Kanülenrohrs in der Trachea auf, wobei das Gelenk in einem Bereich angeordnet ist, der durch die äußeren distalen und proximalen Befestigungsränder des Cuffs begrenzt ist.

Die Begriffe "distal" und "proximal" werden im Sinne der vorliegenden Erfindung aus der Sicht eines behandelnden Arztes verwendet, d.h. dass das proximale Ende der Trachealbeatmungsvorrichtung bzw. des Kanülenrohrs außerhalb des Patientenkörpers liegt, während das distale Ende im eingebrachten Zustand der Trachealbeatmungsvorrichtung im Inneren der Trachea liegt.

Ein Cuff besteht üblicherweise aus einem aufblasbaren, sehr dünnwandigen Schlauch, dessen Endabschnitte außen auf dem Kanülenrohr eng anliegen und dort befestigt sind und der mindestens in einem zentralen Abschnitt gegenüber dem Kanülenrohr ein deutliches Übermaß hat oder auf ein solches Übermaß aufblasbar ist, so dass dieser Abschnitt sich nach dem Einführen der Kanüle in die Trachea aufblasen lässt und mit der Wand der Trachea entlang seines Umfangs abdichtend und mit vorzugsweise geringem Druck in Kontakt kommt. Die Endabschnitte, die im Allgemeinen Ihrerseits Schlauchabschnitte (hier auch "Halsabschnitte" genannt) darstellen, sind typischerweise über ihre Länge und ihren Umfang hinweg mit der Außenwand des Kanülenrohres dicht verklebt oder verschweißt. Die axial äußeren Ränder dieser Endabschnitte bilden dann den vorstehend definierten Bereich für eine bevorzugte Anordnung eines Gelenks.

In einer Ausführungsform weist der befüllbare Cuff einen zentralen Abschnitt und einen sich proximal und/oder distal an den zentralen Abschnitt anschließenden Abschnitt mit einer im Vergleich zu dem zentralen Abschnitt höheren Wandstärke auf, wobei die Wandstärke des sich proximal und/oder distal an den zentralen Abschnitt anschließenden Abschnittes vorzugsweise um 50% bis 300% höher als die Wandstärke des zentralen Abschnittes ist. Die sich an den zentralen Abschnitt proximal und/oder distal anschließenden Abschnitte umschließen gemeinsam mit dem zentralen Abschnitt das Cuffvolumen. Die sich an den zentralen Abschnitt proximal und/oder distal anschließenden Abschnitte können einen Außendurchmesser aufweisen, der von dem zentralen Abschnitt ausgehend in Richtung der Enden des Cuffs abnimmt. In einem Zustand, in dem das Kanülenrohr in eine Trachea eingeführt und der Cuff mit einem Fluid befüllt ist, begrenzt die lokal erhöhte Wandstärke die Auslenkung des distalen Endes des Kanülenrohrs und unterstützt die Gelenkbewegung, sodass ein Anstoßen des Endabschnittes des Rohres an die Innenwand der Trachea verhindert wird.

In einer Ausführungsform sind der proximale und der distale Rohrabschnitt durch einen ringförmigen Spalt mit einer lichten Spaltbreite d voneinander beabstandet und durch das axial luftdurchlässige Gelenk miteinander verbunden, welches den Spalt überbrückt.

In einer Ausführungsform befindet sich der ringförmige Spalt in einem axialen Bereich eines distalen oder proximalen Halsabschnittes eines Cuffs. D. h. der End- bzw. Halsabschnitt des Cuffs verbindet den proximalen und distalen Rohrabschnitt miteinander und wirkt entweder selbst als Gelenk oder umfasst ein entsprechendes Gelenk. Die Wandstärke und Elastizität des Halsabschnittes muss gegebenenfalls an diesen Zweck angepasst sein.

Vorzugsweise ist das Gelenk mit dem proximalen Rohrabschnitt und/oder dem distalen Rohrabschnitt verklebt, verschweißt und/oder angespritzt. Damit durch die Gelenkverbindung kein Atemgas entweichen kann, ist in einer Ausführungsform vorgesehen, dass das Gelenk gasdicht mit dem proximalen Rohrabschnitt und dem distalen Rohrabschnitt verbunden ist. Vorzugsweise ist das Gelenk radial luftundurchlässig.

In einer Ausführungsform weist der distale Rohrabschnitt relativ zu der Achse des proximalen Rohrabschnittes über das Gelenk einen maximalen Biegewinkel in einem Bereich von mindestens +10° bis -10°, vorzugsweise einen Bereich von +25° bis -25°, in beliebiger Richtung relativ zur Kanülenachse auf.

Zweckmäßig ist das Gelenk derart ausgestaltet, dass der Gelenkwinkel zwischen dem proximalen Rohrabschnitt und dem distalen Rohrabschnitt mit einer um mehr als 70 % verringerten Kraft F um 10° veränderbar ist, als sie aufgewendet werden muss, um den proximalen Rohrabschnitt ohne Gelenk um 10° zu verbiegen. Ein derart ausgestaltetes Gelenk ist ausreichend flexibel um einen Schluckprozess nicht zu erschweren und dennoch ausreichend fest, um ein einfaches Einführen der Trachealbeatmungsvorrichtung in eine Trachea zu ermöglichen.

In einer Ausführungsform sind sowohl das Kanülenrohr, d. h. dessen proximale und distale Rohrabschnitte, als auch das Gelenk aus Kunststoff hergestellt, wobei das Gelenk aus einem Kunststoff hergestellt ist, der im Vergleich zu dem Kunststoff des Kanülenrohrs eine geringere Shore-Härte aufweist. Der Kunststoff des Gelenks mit der im Vergleich zu dem Kunststoff des Kanülenrohrs geringeren Shore-Härte ist weicher und daher leichter verformbar und ermöglicht auf diese Weise eine Gelenkbewegung zwischen dem proximalen und distalen Rohrabschnitt. Die im Vergleich zu dem Kunststoff des Gelenks aus einem härteren Kunststoff hergestellten Rohrabschnitte sind hinreichend fest, um einfach in die Trachea eines Patienten eingeführt zu werden. Die aus einem härteren Kunststoff hergestellten Rohrabschnitte haben vorzugsweise eine Shore-A-Härte von 50 bis 98. Der im Vergleich hierzu weichere Kunststoff des Gelenks hat eine um mindestens 50 % geringere Shore-A-Härte.

Die entsprechende Gelenkbeweglichkeit lässt sich auch mit dem gleichen Kunststoffmaterial erzielen, wie es für die proximalen und distalen Rohrabschnitte verwendet wird, das jedoch in dem vorgesehenen Gelenkbereich eine geringere Wandstärke aufweist. Die Wandstärke der proximalen und distalen Rohrabschnitte beträgt vorzugweise zwischen 0,8 mm und 1,8 mm. Demgegenüber beträgt die Wandstärke des Gelenks vorzugsweise zwischen 0,1 mm bis 0,4 mm. Weist das Gelenk einen Schlauchabschnitt auf, so kann die Wandstärke des Schlauchabschnittes deutlich verringert werden. Beispielsweise kann die Wandstärke eines Schlauchabschnittes 10 µm betragen.

In einer Ausführungsform ist das Gelenk in axialer Richtung des Kanülenrohrs elastisch dehnbar und/oder stauchbar, wobei die axiale Spaltbreite d des ringförmigen Spalts zwischen dem proximalen Rohrabschnitt und dem distalen Rohrabschnitt die maximale Stauchbarkeit vorgibt. Die Spaltbreite d ist in einer Ausführungsform 1 mm bis 10 mm, vorzugsweise 2 mm bis 4 mm. Die Stauchbarkeit ist so eingestellt, dass der distale Rohrabschnitt in Richtung des proximalen Rohrabschnittes bewegt wird, wenn der distale Rohrabschnitt mit einer Kraft größer als 0,05 N auf die Trachealwand drückt. Die elastische Stauchbarkeit und Dehnbarkeit sind durch die Verwendung eines entsprechend dünnen und/oder weichen Gelenkmaterials einstellbar. Stößt der distale Rohrabschnitt an die Trachealwand an, verringert das in axiale Richtung des Kanülenrohrs elastisch dehnbare und/oder stauchbare Gelenk eine Belastung der Trachea. Übersteigt die auf die Trachealwand wirkende Kraft z. B. 0,05 N, wird das Gelenk gestaucht und der Abstand zwischen dem distalen Rohrabschnitt und dem proximalen Rohrabschnitt wird verringert Hat die Kraft eine Komponente, die einen von 0° verschiedenen Winkel mit der Mittelachse des distalen Rohrabschnittes einschließt, so dass sie nicht senkrecht auf die Wand der Trachea wirkt, kann der distale Rohrabschnitt gegenüber dem proximalen Rohrabschnitt abknicken was auc durch die Stauchbarkeit erleichtert wird..

Wenn eine solches dehn- und/oder stauchbares Gelenk innerhalb eines zentralen Cuff angeordnet ist, dämpft der Cuff die Relativbewegung zwischen dem proximalen Rohrabschnitt und dem distalen Rohrabschnitt. Werden der proximale Rohrabschnitt und der distale Rohrabschnitt aufeinander zubewegt, sinkt das Cuffvolumen und der Cuffdruck steigt. Vergrößert sich der Abstand zwischen dem proximalen Rohrabschnitt und dem distalen Rohrabschnitt dehnt sich das Volumen des Cuffs aus und der Cuffdruck sinkt. Umgekehrt kann man über eine Einstellung des Cuffdrucks innerhalb gewisser Grenzen auch die Dehnung bzw. das Maß des elastischen Zusammenziehens dehnbarer elastischer Schlauchabschnitte des Gelenks einstellen.

Der Cuffdruck ermöglicht eine Pufferung des Stauchens der Kanüle in axialer Richtung des Kanülenrohrs. Das Gelenk und der das Gelenk umgebende, unter leichtem Überdruck mit Gas befüllte Cuff wirken wie ein Stoßdämpfer, der Stöße auf die Trachealbeatmungsvorrichtung aufnimmt und verringert. Auf diese Weise können Schädigungen der Trachealwand durch das distale Kanülenende reduziert und der Schluckvorgang erleichtert werden. Vorzugsweise wird ein Cuff mit Hilfe von Luft mit einem Druck von 15 mbar bis 30 mbar beaufschlagt. Der radial außenliegend zu dem ringförmigen Spalt angeordnete Cuff bewirkt eine Rückstellkraft in die Ausgangslage des Gelenks. Zugleich verhindert der befüllte Cuff, dass die an dem Spalt befindlichen Enden des proximalen und des distalen Rohrabschnittes gegen die Trachea drücken und diese schädigen. Der Cuff polstert den ringförmigen Spalt und das Gelenk der Trachealbeatmungsvorrichtung gegenüber der Trachea ab, so dass eine bündige Fixierung eines das Gelenk bildenden Schlauchabschnittes an den jeweiligen Rohrabschnitten nicht erforderlich ist.

In einer Ausführungsform ist das Gelenk ein Schlauchabschnitt, der auf der einen Seite eines ringförmigen Spalts zwischen dem proximalen und dem distalen Rohrabschnitt an dem proximalen Rohrabschnitt und auf der anderen Seite des Spalts an dem distalen Rohrabschnitt befestigt ist. Vorzugsweise weist der Kunststoff des Schlauchabschnitts eine im Vergleich zu dem Kunststoff des Kanülenrohrs geringere Shore-Härte auf. Der Schlauchabschnitt aus dem weicheren Kunststoff ist elastisch verformbar, sodass die Spaltbreite d, d.h. der lichte Abstand zwischen dem proximalen Rohrabschnitt und dem distalen Rohrabschnitt, auf gegenüberliegenden Seiten der Rohrabschnitte in unterschiedlichem Maße und/oder in unterschiedlicher Richtung veränderbar ist. Wird der Schlauchabschnitt einseitig gestaucht oder gedehnt, verkleinert oder vergrößert sich die Spaltbreite d des ringförmigen Spalts entsprechend auf einer Seite, sodass der proximale Rohrabschnitt und der distale Rohrabschnitt relativ zueinander verschwenkt werden.

In einer Ausführungsform ist der Schlauchabschnitt beidseitig des ringförmigen Spalts an dem proximalen Rohrabschnitt und dem distalen Rohrabschnitt befestigt. Vorzugsweise ist der Schlauchabschnitt stumpf auf die Stirnseiten des proximalen und des distalen Rohrabschnittes aufgesetzt verklebt, verschweißt oder angespritzt. In einer anderen Ausführungsform weisen die einander zugewandten Enden der Rohrabschnitte einen Falz zur bündigen und glatten Aufnahme des komplementär ausgestalten Schlauchabschnittes auf. Der Schlauchabschnitt verbindet wie eine Muffe den proximalen Rohrabschnitt und den distalen Rohrabschnitt für die Durchleitung von Atemgas.

In einer Ausführungsform weist das Gelenk zusätzlich zu dem zuvor beschriebenen Schlauchabschnitt einen zweiten Schlauchabschnitt auf, der konzentrisch zu dem ersten Schlauchabschnitt angeordnet ist, wobei der erste Schlauchabschnitt radial außenliegend mit dem proximalen Rohrabschnitt und dem distalen Rohrabschnitt verbunden und der zweite Schlauchabschnitt radial innenliegend mit dem proximalen Rohrabschnitt und dem distalen Rohrabschnitt verbunden ist. Die zwei Schlauchabschnitte verbinden den proximalen Rohrabschnitt und den distalen Rohrabschnitt sowohl auf der Innenseite des Kanülenrohrs, d.h. im zentralen Lumen des Kanülenrohrs, als auch auf der Außenseite des Kanülenrohrs. Der erste Schlauchabschnitt und/oder der zweite Schlauchabschnitt überspannen den ringförmigen Spalt und sind beidseitig des ringförmigen Spalts an dem proximalen Rohrabschnitt und dem distalen Rohrabschnitt befestigt. Beispielsweise ist der eine oder sind beide Schlauchabschnitte durch Verkleben, Verschweißen oder Anspritzen an den proximalen und distalen Rohrabschnitten befestigt.

Erfindungsgemäß weist der eine Schlauchabschnitt des Gelenks, oder falls das Gelenk aus zwei Schlauchabschnitten besteht, beide Schlauchabschnitte, eine geringere Wandstärke als die mittlere Wandstärke des proximalen Rohrabschnitts im Anschlussbereich des Gelenks auf. Beispielsweise ist die mittlere Wandstärke des ersten Schlauchabschnittes und/oder des zweiten Schlauchabschnittes ¼ der mittleren Wandstärke des proximalen Rohrabschnitts im Anschlussbereich des Gelenks.

In einer Ausführungsform ist der Schlauchabschnitt stumpf mit den Enden der proximalen und distalen Rohrabschnitte verbunden. Somit füllt der Schlauchabschnitt den ringförmigen Spalt zwischen dem proximalen Rohrabschnitt und dem distalen Rohrabschnitt aus. Der Schlauchabschnitt kann sich nahtlos an den proximalen Rohrabschnitt und den distalen Rohrabschnitt anschließen. Der Schlauchabschnitt hat in seinem proximalen Anschlussbereich eine Wandstärke, die der Wandstärke des proximalen Rohrabschnittes entspricht, und in seinem distalen Anschlussbereich eine Wandstärke, die der Wandstärke des distalen Rohrabschnittes entspricht. In einer etwas modifizierten Ausführungsform hat der der Schlauchabschnitt eine größere Wandstärke als der proximale Rohrabschnitt und der distale Rohrabschnitt, mit je einer ringförmigen Nut in den Stirnseiten des Schlauchabschnittes für die Aufnahme der Enden des proximalen und distalen Rohrabschnitte in diesen Nuten. Die sich über den Spalt in axialer Länge des Kanülenrohrs hinaus und in den Bereich des proximalen und distalen Rohabschnittes erstreckenden Abschnitte des Schlauchabschnittes sind beidseitig des Spaltes an dem proximalen Rohrabschnitt und dem distalen Rohrabschnitt befestigt, vorzugsweise verklebt, verschweißt oder angespritzt.

Um das Gelenk zu stabilisieren, weist das Gelenk in einer Ausführungsform eine Mehrzahl von Kunststoffstegen, vorzugsweise zwei diametral gegenüberliegend angeordnete Kunststoffstege auf, die den ringförmigen Spalt überbrücken und den proximalen Rohrabschnitt mit dem distalen Rohrabschnitt verbinden. Vorzugsweise sind die Kunststoffstege gleichmäßig in Umfangsrichtung des ringförmigen Spalts verteilt angeordnet. Die Anordnung der Kunststoffstege ist geeignet, die Freiheitsgrade des Gelenks einzuschränken. Weist das Gelenk beispielsweise zwei Kunststoffstege auf, die in einem Winkelabstand von 180° oder auch etwas weniger in Umfangsrichtung des ringförmigen Spalts angeordnet sind, weist das Gelenk, ähnlich einem Scharniergelenk, eine bevorzugte Schwenkrichtung in etwa parallel zu den Kunststoffstegen auf. In einer Ausführungsform sind die Stege seitlich angeordnet, sodass mit dem Gelenk der Biegewinkel des Kanülenrohrs vergrößert oder verkleinert werden kann, zusätzlich eine Verbiegbarkeit in seitliche Richtung reduziert wird. Die Kunststoffstege können aus dem gleichen Kunststoff wie die Rohrabschnitte oder aus einem im Vergleich hierzu weicheren Kunststoff gefertigt sein. Gleichzeitig ist das Gelenk durch die Stege gegenüber axialen Kräften versteift.

In einer Ausführungsform ist die axiale Spaltbreite d des ringförmigen Spalts 1 mm bis 10 mm, vorzugsweise 3 mm bis 4 mm. Derartige Spaltbreiten verhindern, dass sich bei einer maximalen Gelenkbewegung das zentrale Lumen des Kanülenrohrs verschließt und die Atmung durch die Trachealbeatmungsvorrichtung wesentlich erschwert wird.

In einer Ausführungsform wird das Gelenk aus einem Wandabschnitt des Kanülenrohres gebildet der den proximalen Rohrabschnitt vom distalen Rohrabschnitt trennt, wobei dieser Wandabschnitt eine reduzierte Wandstärke aufweist.

Weitere Vorteile, Merkmale und Anwendungsmöglichkeiten der vorliegenden Erfindung werden anhand der vorliegenden Beschreibung einer Ausführungsform und der dazugehörigen Figuren deutlich, wobei gleiche Bezugszeichen auf gleiche Elemente verweisen. Es zeigen:
- Figur 1:: einen Längsschnitt durch eine Trachealbeatmungsvorrichtung gemäß einer Ausführungsform der vorliegenden Erfindung,
- Figur 2:: eine Detailansicht des Gelenks der Ausführungsform gemäß Figur 1,
- Figur 3:: einen Teilschnitt des Gelenks einer weiteren Ausführungsform der vorliegenden Erfindung,
- Figur 4:: einen Teilschnitt des Gelenks einer weiteren Ausführungsform der vorliegenden Erfindung,
- Figur 5:: eine perspektivische Detailansicht eines Gelenks einer weiteren Ausführungsform der vorliegenden Erfindung,
- Figur 6:: eine Schnittansicht der Figur 5, und
- Figur 7:: einen Längsschnitt durch eine Trachealbeatmungsvorrichtung gemäß einer weiteren Ausführungsform der vorliegenden Erfindung.

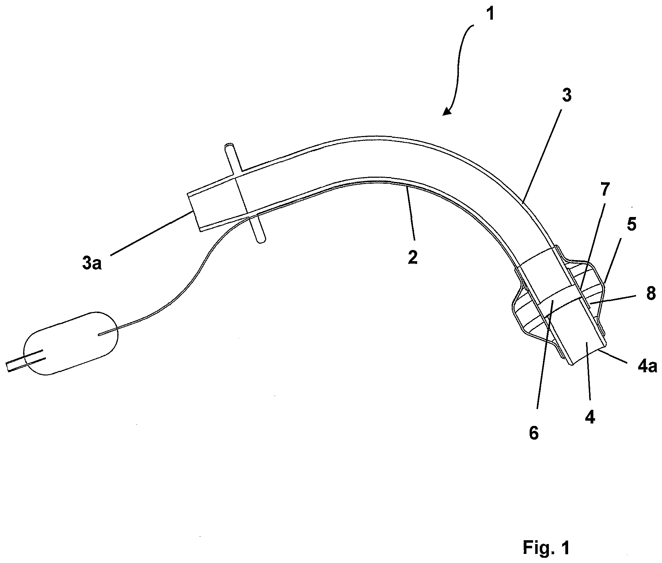

In der Figur 1 ist ein Längsschnitt durch eine Trachealbeatmungsvorrichtung 1 gemäß einer erfindungsgemäßen Ausführungsform dargestellt. Die Trachealbeatmungsvorrichtung 1 hat ein Kanülenrohr 2 aus Kunststoff, welches einen proximalen Rohrabschnitt 3 mit einer proximalen Öffnung 3a und einen distalen Rohrabschnitt 4 mit einer distalen Öffnung 4a aufweist. Auf seinem Außenumfang weist das Kanülenrohr 2 einen Cuff 5 auf, welcher nahe dem distalen Ende des Kanülenrohrs 2 angeordnet und in seinem befüllten Zustand gezeigt ist. Der Abstand des befüllbaren Cuffabschnitts von dem distalen Ende des Kanülenrohrs beträgt 3 mm bis 8 mm.

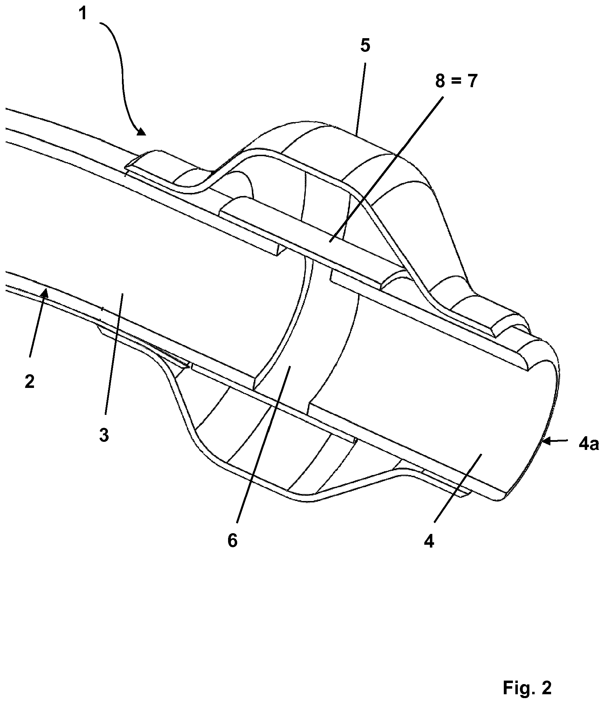

An einer von dem Cuff 5 überdeckten axialen Position und radial innerhalb des Cuffs 5 trennt ein ringförmiger Spalt 6 den proximalen Rohrabschnitt 3 und den distalen Rohrabschnitt 4. Der axiale Abstand zwischen dem proximalen Rohrabschnitt 3 und dem distalen Rohrabschnitt 4 entspricht der Breite d des ringförmigen Spaltes 6. Wie in der Detailansicht der Figur 2 gut erkennbar ist, sind der proximale Rohrabschnitt 3 und der distale Rohrabschnitt 4 durch ein Gelenk 7 miteinander verbunden. Das Gelenk 7 ist ein Schlauchabschnitt 8, der radial außen an dem Kanülenrohr 2 angeordnet und den proximalen Rohrabschnitt 3 und den distalen Rohrabschnitt 4 verbindet. Der Schlauchabschnitt 8 überspannt den ringförmigen Spalt 6 und ist beiderseits des Spalts 6 an dem proximalen Rohrabschnitt 3 und dem distalen Rohrabschnitt 4 befestigt. Der Schlauchabschnitt 8 ist mit dem proximalen Rohrabschnitt 3 und dem distalen Rohrabschnitt 4 verklebt, verschweißt oder angespritzt.

Der Schlauchabschnitt 8 ist aus einem im Vergleich zu dem Kunststoff, aus dem der proximale Rohrabschnitt 3 und der distale Rohrabschnitt 4 hergestellt sind, weicheren Kunststoff gefertigt. Die Wandstärke des Schlauchabschnittes 8 ist kleiner als die mittlere Wandstärke des proximalen Rohrabschnitts 3 im Anschlussbereich des Gelenks 7.

In dem dargestellten befüllten Zustand polstert der Cuff 5 das Gelenk 7 und verhindert so, dass, wenn die Trachealbeatmungsvorrichtung 1 in die Trachea eines Patienten eingeführt ist, das Gelenk 7 oder sich die an den ringförmigen Spalt 6 anschließenden Enden des proximalen bzw. distalen Rohrabschnittes 3, 4 gegen die Wand der Trachea reiben, dass Schädigungen entstehen.

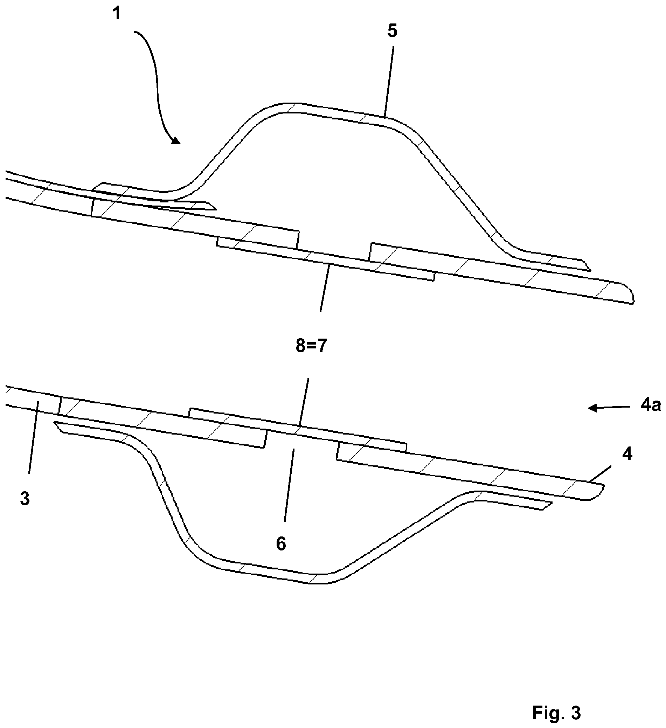

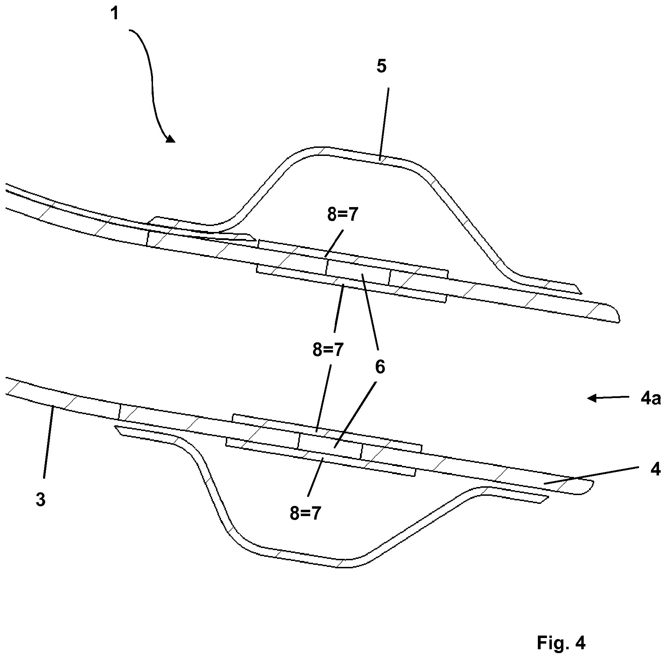

Die Figuren 3 und 4 zeigen alternative Ausführungsformen der vorliegenden Erfindung in einer Schnittansicht. Der einzige Unterschied im Vergleich zu der Ausführungsform aus den Figuren 1 und 2 ist die Ausgestaltung des Gelenks 7.

Bei der Ausführungsform gemäß der Figur 3 besteht das Gelenk 7 aus einem Schlauchabschnitt 8 ("Muffe"), der auf der Innenseite des Kanülenrohrs 2 angeordnet ist und den proximalen Rohrabschnitt 3 und den distalen Rohrabschnitt 4 verbindet. Der Schlauchabschnitt 8 erstreckt sich über den ringförmigen Spalt 6 hinaus und ist beidseitig des Spaltes 6 mit dem proximalen Rohrabschnitt 3 und dem distalen Rohrabschnitt 4 auf der Innenseite des Kanülenrohrs 2 befestigt. Der Kunststoff des Schlauchabschnittes 8 weist eine geringere Shore-Härte auf als der Kunststoff, aus dem der proximale und der distale Rohrabschnitt 3, 4 hergestellt sind.

Das Gelenk 7 der Ausführungsformen gemäß den Figuren 1 bis 3 weist genau einen Schlauchabschnitt 8 auf, der den proximalen Rohrabschnitt 3 und den distalen Rohrabschnitt 4 radial gasdicht verbindet.

Das Gelenk 7 gemäß der in der Figur 4 gezeigten Ausführungsform weist genau zwei Schlauchabschnitte 8 auf, von denen ein erster Schlauchabschnitt 8 radial innen und ein zweiter Schlauchabschnitt 8 radial außen an dem Kanülenrohr 2 angeordnet sind. Beide Schlauchabschnitte 8 sind aus einem weicheren Kunststoff hergestellt als der proximale und der distale Rohrabschnitt 3, 4.

Wie bei den zuvor beschriebenen Ausführungsformen erstrecken sich die Schlauchabschnitte 8 über den ringförmigen Spalt 6 und sind beidseitig an dem proximalen Rohrabschnitt 3 und dem distalen Rohrabschnitt 4 befestigt, sodass diese Schlauchabschnitte ein axial gasdurchlässiges Gelenk 7 bilden. Der ringförmige Spalt 6 ist nicht gefüllt, sodass der ringförmige Bereich zwischen den Schlauchabschnitten 8 einen Hohlraum bildet. Das Gelenk 7 ist radial luftundurchlässig.

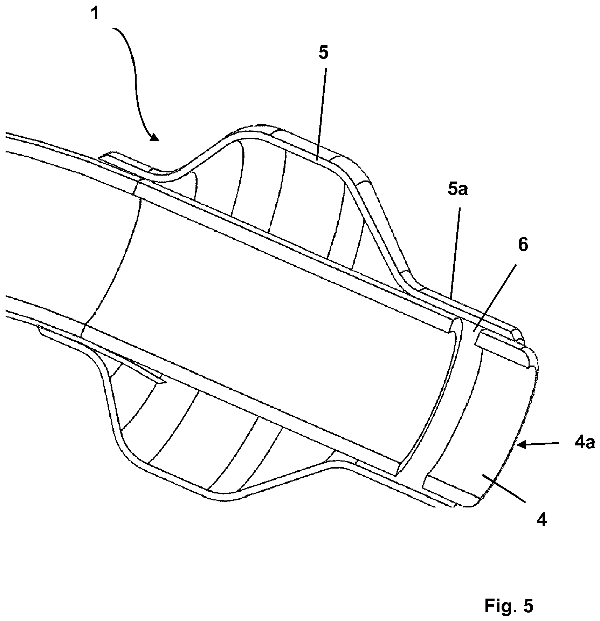

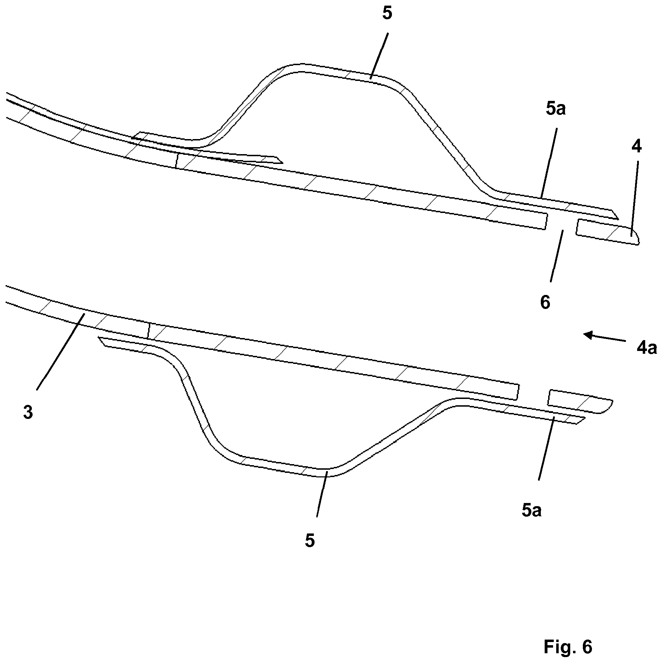

Die Figuren 5 und 6 zeigen eine weitere Ausführungsform einer erfindungsgemäßen Trachealbeatmungsvorrichtung 1 in einer Detailansicht. Die Ausführungsform der Figuren 5 und 6 unterscheidet sich von den zuvor beschriebenen Ausführungsformen lediglich in der Anordnung des ringförmigen Spaltes 6 und der Ausgestaltung des axial gasdurchlässigen Gelenks 7, welches den proximalen Rohrabschnitt 3 und den distalen Rohrabschnitt 4 gelenkig verbindet. Der ringförmige Spalt 6 ist in einem axialen Bereich angeordnet, der von einem distalen Halsabschnitt 5a des Cuffs 5 überdeckt ist. Das Gelenk 7 wird bei dieser Ausführungsform durch den Halsabschnitt 5a des Cuffs 5 gebildet. Der Halsabschnitt 5a überspannt radial außenliegend den ringförmigen Spalt 6 und ist beidseitig des ringförmigen Spaltes 6 mit dem proximalen Rohrabschnitt 3 und dem distalen Rohrabschnitt 4 gasdicht verklebt. Der Cuff 5 ist einschließlich des Halsabschnittes 5a aus einem weicheren Kunststoff hergestellt als der Kunststoff aus dem der proximale und der distale Rohrabschnitt 3, 4 geformt sind.

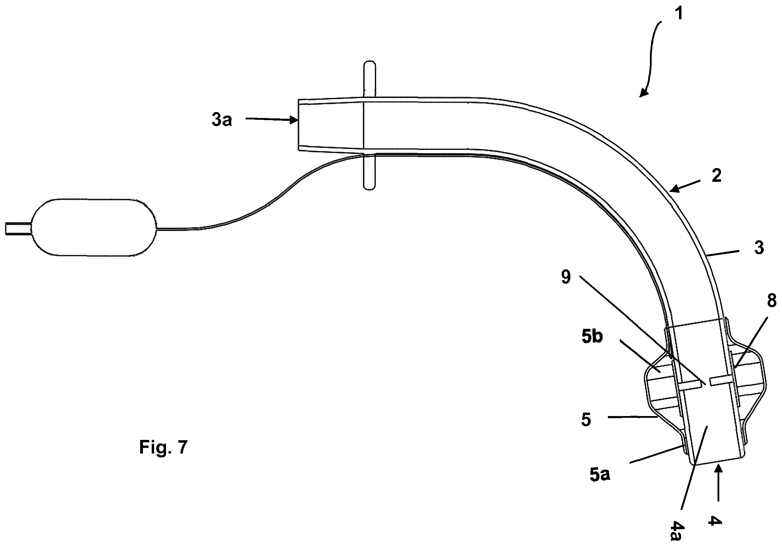

In der Figur 7 ist ein Längsschnitt durch eine Trachealbeatmungsvorrichtung 1 einer weiteren Ausführungsform der vorliegenden Erfindung gezeigt. Die Trachealbeatmungsvorrichtung 1 unterscheidet sich von den Trachealbeatmungsvorrichtungen der zuvor beschriebenen Ausführungsformen in der Anordnung des ringförmigen Spaltes 6, der den proximalen Rohrabschnitt 3 und den distalen Rohrabschnitt 4 trennt, sowie in der Ausgestaltung des Gelenks 7. Der ringförmige Spalt 6 ist in einem axialen Bereich des Kanülenrohrs 2 angeordnet, der von dem Cuff 5, genauer von dem befüllbaren Abschnitt 5b des Cuffs 5, überdeckt wird. Auf der radial außenliegenden Seite des Kanülenrohrs 2 ist ein Schlauchabschnitt 8 angeordnet, der den ringförmigen Spalt 6 überspannt und beidseitig des Spaltes 6 mit dem proximalen Rohrabschnitt 3 und dem distalen Rohrabschnitt 4 verklebt ist. Der Schlauchabschnitt 8 bildet das Gelenk 7, dass die Rohrabschnitte 3, 4 miteinander gelenkig verbindet. Der Schlauchabschnitt 8 ist aus einem weicheren Kunststoff hergestellt als der Kunststoff aus dem die Rohrabschnitte 3, 4 gefertigt sind. In dem ringförmigen Spalt 6 sind diametral gegenüberliegend zwei Verstärkungsstreben 9 angeordnet, die den proximalen Rohrabschnitt 3 und den proximalen Rohrabschnitt 4 zusätzlich zu dem Schlauchabschnitt 8 verbinden. Die Verstärkungsstreben 9 beschränkt die Anzahl der Freiheitsgrade des Gelenks 7 und bewirken, dass das Gelenk 7, ähnlich einem Scharniergelenk, bewegbar ist.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarerer Merkmalskombinationen wird hier nur der Kürze und der Lesbarkeit der Beschreibung wegen verzichtet.

Während die Erfindung im Detail in den Zeichnungen und der vorangehenden Beschreibung dargestellt und beschreiben wurde, erfolgt diese Darstellung und Beschreibung lediglich beispielhaft und ist nicht als Beschränkung des Schutzbereichs gedacht, so wie er durch die Ansprüche definiert wird. Die Erfindung ist nicht auf die offenbarten Ausführungsformen beschränkt.

Abwandlungen der offenbarten Ausführungsformen sind für den Fachmann aus den Zeichnungen, der Beschreibung und den beigefügten Ansprüchen offensichtlich. In den Ansprüchen schließt das Wort "aufweisen" nicht andere Elemente oder Schritte aus, und der unbestimmte Artikel "eine" oder "ein" schließt eine Mehrzahl nicht aus. Die bloße Tatsache, dass bestimmte Merkmale in unterschiedlichen Ansprüchen beansprucht sind, schließt ihre Kombination nicht aus. Bezugszeichen in den Ansprüchen sind nicht als Beschränkung des Schutzbereichs gedacht.

### Bezugszeichenliste

- 1: Trachealbeatmungsvorrichtung
- 2: Kanülenrohr
- 3: proximaler Rohrabschnitt
- 3a: proximale Öffnung
- 4: distaler Rohrabschnitt
- 4a: distale Öffnung
- 5: Cuff
- 5a: distaler Halsabschnitt
- 5b: befüllbarer Abschnitt
- 6: ringförmiger Spalt
- 7: Gelenk
- 8: Schlauchabschnitt
- 9: Kunststoffstege

## Patentansprüche

1. Trachealbeatmungsvorrichtung (1), insbesondere Tracheostomiekanüle oder Endotrachealtubus, mit einem Kanülenrohr (2), welches einen proximalen Rohrabschnitt (3) mit einer proximalen Öffnung (3a) und einen distalen Rohrabschnitt (4) mit einer distalen Öffnung (4a) aufweist, wobei der proximale Rohrabschnitt (3) und der distale Rohrabschnitt (4) durch ein axial luftdurchlässiges Gelenk (7) miteinander verbunden sind, welches mindestens eine begrenzte Bewegbarkeit und insbesondere Verschwenkbarkeit der Achse des distalen Rohrabschnittes (4) bezüglich der Achse des proximalen Rohrabschnitts (3) ermöglicht wobei das Kanülenrohr (2) auf seinem äußeren Umfang einen befüllbaren Cuff (5) zur Abdichtung und weichen Fixierung des Kanülenrohrs in der Trachea aufweist, wobei das Gelenk (7) in einem Bereich angeordnet ist, der durch die äußeren distalen und proximalen Befestigungsränder des Cuffs begrenzt ist, **dadurch gekennzeichnet, dass** das Gelenk als Schlauchabschnitt ausgebildet ist, wobei der Schlauchabschnitt im Vergleich zu den proximalen und distalen Rohrabschnitten eine geringere Wandstärke aufweist.

2. Trachealbeatmungsvorrichtung (1) nach Anspruch 1, **dadurch gekennzeichnet, dass** der befüllbare Cuff (5) einen zentralen Abschnitt und einen sich proximal und/oder distal an den zentralen Abschnitt anschließenden Abschnitt mit einer im Vergleich zu dem zentralen Abschnitt höheren Wandstärke aufweist, wobei die Wandstärke des sich proximal und/oder distal an den zentralen Abschnitt anschließenden Abschnitt vorzugsweise um 50% bis 300% höher als die Wandstärke des zentralen Abschnittes ist.

3. Trachealbeatmungsvorrichtung (1) nach Anspruch 1 oder 2, **dadurch gekennzeichnet, dass** der proximale und der distale Rohrabschnitt (3, 4) durch einen ringförmigen Spalt (6) mit einer lichten Spaltbreite d voneinander beabstandet und durch das Gelenk (7) miteinander verbunden sind, welches den Spalt (6) überbrückt, wobei der Cuff (5) einen nicht aufblasbaren distalen Halsabschnitt (5a) aufweist und das Gelenk, vorzugsweise in Form eines von mindestens einem Verbindungselement überbrückten ringförmigen Spaltes (6), an einer von dem Halsabschnitt (5a) des Cuffs (5) überdeckten axialen Position und radial innerhalb dieses Halsabschnittes (5a) angeordnet ist.

4. Trachealbeatmungsvorrichtung (1) nach Anspruch 3, **dadurch gekennzeichnet, dass** das Gelenk (7) aus einem Teil der Wand eines Halsabschnittes (5a) des Cuffs (5), gebildet ist.

5. Trachealbeatmungsvorrichtung (1) nach einem der Ansprüche 1 bis 4, **dadurch gekennzeichnet, dass** das Gelenk (7) in axialer Richtung des Kanülenrohrs (2) elastisch dehnbar und/oder stauchbar ist, wobei vorzugsweise das Gelenk durch einen dehnbaren und/oder stauchbaren Schlauchabschnitt gebildet wird.

6. Trachealbeatmungsvorrichtung (1) nach Anspruch 5, **dadurch gekennzeichnet, dass** das Gelenk mindestens einen Teil der Innenwand des aufblasbaren Cuffabschnittes bildet.

7. Trachealbeatmungsvorrichtung (1) nach Anspruch 5 oder 6, **dadurch gekennzeichnet, dass** die Änderung der axialen Spaltbreite d eines ringförmigen Spalts zwischen dem proximalen Rohrabschnitt (3) und dem distalen Rohrabschnitt (4) bei einer elastischen Verformung des Gelenks 1 mm bis 10 mm, vorzugsweise 2 mm bis 4 mm, beträgt.

8. Trachealbeatmungsvorrichtung (1) nach einem der Ansprüche 1 bis 7, **dadurch gekennzeichnet, dass** der distale Rohrabschnitt (4) relativ zur Achse des proximalen Rohrabschnitts (3) über das Gelenk (7) einen maximalen Biegewinkel in einem Bereich von mindestens +10° bis -10, vorzugsweise einen Bereich von +25° bis -25°, aufweist.

9. Trachealbeatmungsvorrichtung (1) nach einem der Ansprüche 1 bis 8, **dadurch gekennzeichnet, dass** der Gelenkwinkel zwischen dem proximalen Rohrabschnitt (3) und dem distalen Rohrabschnitt (4) mit einer um mindestens 70 % geringeren Kraft F um 10° veränderbar ist, als sie aufgewendet werden muss, um den proximalen Rohrabschnitt (3) ohne Gelenk um 10° zu verbiegen.

10. Trachealbeatmungsvorrichtung (1) nach einem der Ansprüche 1 bis 9, wobei das Kanülenrohr (2) aus Kunststoff hergestellt ist, **dadurch gekennzeichnet, dass** das Gelenk (7) ein Schlauchabschnitt (8) ist, der aus einem Kunststoff mit einer im Vergleich zu dem Kunststoff des Kanülenrohrs (2) geringeren Shore-Härte hergestellt ist und den proximalen Rohrabschnitt (3) mit dem distalen Rohrabschnitt (4) verbindet, wobei vorzugsweise das Gelenk (7) einen zweiten Schlauchabschnitt (8) aufweist, der konzentrisch zu dem ersten Schlauchabschnitt (8) angeordnet ist, wobei der erste Schlauchabschnitt (8) radial außenliegend mit dem proximalen Rohrabschnitt (3) und dem distalen Rohrabschnitt (4) verbunden ist und der zweite Schlauchabschnitt (8) radial innenliegend mit dem proximalen Rohrabschnitt (3) und dem distalen Rohrabschnitt (4) verbunden ist.

11. Trachealbeatmungsvorrichtung (1) nach Anspruch 10, **dadurch gekennzeichnet, dass** der erste Schlauchabschnitt und/oder der zweite Schlauchabschnitt mit dem proximalen und dem distalen Schlauchabschnitt überlappen, vorzugsweise beträgt der Überlappungsbereich auf jeder Seite des Spaltes 2 mm bis 6 mm.

12. Trachealbeatmungsvorrichtung (1) nach Anspruch 10 oder 11, **dadurch gekennzeichnet, dass** der erste Schlauchabschnitt (8) und/oder der zweite Schlauchabschnitt (8) eine im Vergleich zu der mittleren Wandstärke des proximalen Rohrabschnitts (3) im Anschlussbereich des Gelenks (7) reduzierte Wandstärke aufweist, wobei vorzugsweise die reduzierte Wandstärke 0,1 mm bis 0,4 mm beträgt.

13. Trachealbeatmungsvorrichtung (1) nach Anspruch 5 bis 10, **dadurch gekennzeichnet, dass** der Schlauchabschnitt (8) den proximalen Rohrabschnitt (3) und den distalen Rohrabschnitt (4) bündig miteinander verbindet.

14. Trachealbeatmungsvorrichtung nach einem der Ansprüche 1 bis 13, **dadurch gekennzeichnet, dass** das Gelenk (7) eine Mehrzahl von Kunststoffstegen (9) aufweist, die den proximalen Rohrabschnitt (3) mit dem distalen Rohrabschnitt (4) verbinden.

15. Trachealbeatmungsvorrichtung (1) nach einem der Ansprüche 1 bis 14, **dadurch gekennzeichnet, dass** die axiale Spaltbreite d des ringförmigen Spalts (6) 1 mm bis 10 mm, vorzugsweise 2 mm bis 4 mm, beträgt, wobei vorzugsweise das Gelenk (7) mit dem proximalen Rohrabschnitt (3) und/oder dem distalen Rohrabschnitt (4) verklebt, verschweißt und/oder angespritzt ist.

## Claims

1. Tracheal ventilation device (1), in particular tracheostomy cannula or endotracheal tube, comprising a cannula tube (2) which has a proximal tube portion (3) with a proximal opening (3a) and a distal tube portion (4) having a distal opening (4a), **characterised in that** the proximal tube portion (3) and the distal tube portion (4) are connected to one another by way of an axially air-permeable joint (7) that facilitates at least restricted mobility and, in particular, pivotability of the axis of the distal tube portion (4) with respect to the axis of the proximal tube portion (3), wherein the cannula tube (2) has a fillable cuff (5) on its outer periphery for sealing and soft fixing the cannula tube in the trachea, wherein the joint is arranged in a region, which is delimited by the outer distal and proximal fastening edges of the cuff, **characterised in that** the joint is formed as a tube portion, wherein the tube portion with respect to the proximal and distal tube portions has a smaller wall thickness.

2. Tracheal ventilation device (1) according to Claim 1, **characterised in that** the fillable cuff (5) has a central portion and a portion which adjoins the central portion proximally and/or distally and has a greater wall thickness than the central portion, wherein the wall thickness of the portion adjoining the central portion proximally and/or distally is preferably 50% to 300% greater than the wall thickness of the central portion.

3. Tracheal ventilation device (1) according to Claim 1 or 2, **characterised in that** the proximal and the distal tube portions (3, 4) are spaced apart from one another by an annular gap (6) having a clear gap width d and are connected to one another by way of the joint (7) which bridges the gap (6), wherein the cuff (5) comprises a non-inflatable distal neck portion (5a) and the joint, preferably in the form of an annular gap (6) bridged by at least one connecting element, is arranged in an axial position covered by the neck portion (5a) of the cuff (5) and radially inside said neck portion (5a).

4. Tracheal ventilation device (1) according to Claim 3, **characterised in that** the joint (7) is formed from a part of the wall of a neck section (5a) of the cuff (5).

5. Tracheal ventilation device (1) according to any one of Claims 1 to 4, **characterised in that** the joint (7) is elastically expandable and/or compressible in the axial direction of the cannula tube (2), wherein preferably the joint is formed by an expandable and/or compressible sleeve portion.

6. Tracheal ventilation device (1) according to Claim 5, **characterised in that** the joint forms at least a part of the inner wall of the inflatable cuff portion.

7. Tracheal ventilation device (1) according to any one of Claims 5 or 6, **characterised in that** the change in the axial gap width d of an annular gap between the proximal tube portion (3) and the distal tube portion (4) in the event of an elastic deformation of the joint is 1 mm to 10 mm, preferably 2 mm to 4 mm.

8. Tracheal ventilation device (1) according to any one of Claims 1 to 7, **characterised in that** the distal tube portion (4) has a maximum bending angle relative to the axis of the proximal tube portion (3) by way of the joint (7) in the region of at least +10° to -10°, preferably in the region of +25° to -25°.

9. Tracheal ventilation device (1) according to any one of Claims 1 to 8, **characterised in that** the joint angle between the proximal tube portion (3) and the distal tube portion (4) can be changed by 10° using a force F, which is reduced by at least 70%, as it has to be rotated in order to bend the proximal tube portion (3) by 10° without a joint.

10. Tracheal ventilation device (1) according to any one of Claims 1 to 9, wherein the cannula tube (2) is made of plastic, **characterised in that** the joint (7) is a sleeve portion (8) which is made of a plastic with a lower shore hardness than the plastic of the cannula tube (2) and connects the proximal tube portion (3) to the distal tube portion (4), wherein preferably the joint (7) comprises a second sleeve portion (8) which is arranged concentrically with respect to the first sleeve portion (8), wherein the first sleeve portion (8) is connected to the proximal tube portion (3) and the distal tube portion (4) radially on the outside, and the second sleeve portion (8) is connected to the proximal tube portion (3) and the distal tube portion (4) radially on the inside.

11. Tracheal ventilation device (1) according to Claim 10, **characterised in that** the first sleeve portion and/or the second sleeve portion overlap with the proximal and the distal tube portion, wherein preferably, the overlap region on each side of the gap is 2 mm to 6 mm.

12. Tracheal ventilation device (1) according to Claim 10 or 11, **characterised in that** the first sleeve portion (8) and/or the second sleeve portion (8) has a reduced wall thickness compared with the average wall thickness of the proximal tube portion (3) in the connection region of the joint (7), wherein preferably the reduced wall thickness is 0.1 mm to 0.4 mm.

13. Tracheal ventilation device (1) according to any one of Claims 5 to 10, **characterised in that** the sleeve portion (8) connects the proximal tube portion (3) and the distal tube portion (4) to one another in a flush manner.

14. Tracheal ventilation device (1) according to any one of Claims 1 to 13, **characterised in that** the joint (7) has a plurality of plastic struts (9) which connect the proximal tube portion (3) to the distal tube portion (4).

15. Tracheal ventilation device (1) according to any one of Claims 1 to 14, **characterised in that** the axial gap width d of the annular gap (6) is 1 mm to 10 mm, preferably 2 mm to 4 mm, wherein preferably the joint (7) is adhesively bonded, welded and/or injection-moulded to the proximal tube portion (3) and/or the distal tube portion (4).

## Revendications

1. Dispositif de respiration artificielle trachéale (1), en particulier canule de trachéostomie ou tube endotrachéal, avec un tube de canule (2) en plastique présentant une partie de tube proximale (2a) munie d'une ouverture proximale (3a) et une partie de tube distale (4) munie d'une ouverture distale (4a), dans lequel la partie de tube proximale (3) et la partie de tube distale (4) sont reliées grâce à une articulation (7) laissant passer l'air de manière axiale et permettant au moins une mobilité, et en particulier une capacité à pivoter, de l'axe de la partie de tube distale (4) par rapport à l'axe de la partie de tube proximale (3), dans lequel le tube de canule (2) présente sur sa périphérie extérieure un manchon (5) pouvant être rempli et permettant une étanchéité et une fixation souple du tube de canule dans la trachée, dans lequel l'articulation (7) est agencée dans une région qui est limitée par les bords de fixation externes distal et proximal du manchon, **caractérisé en ce que** l'articulation est réalisée sous forme de partie de tuyau flexible, dans lequel la partie de tuyau flexible présente une épaisseur de paroi inférieure à celle des parties de tube proximale et distale.

2. Dispositif de respiration artificielle trachéale (1) selon la revendication 1, **caractérisé en ce que** le manchon (5) pouvant être rempli présente une partie centrale et une partie se raccordant de manière proximale et/ou distale à la partie centrale avec une épaisseur de paroi supérieure à celle de la partie centrale, dans lequel l'épaisseur de paroi de la partie se raccordant de manière proximale et/ou distale à la partie centrale est de manière préférée supérieure de 50 % à 300 % à l'épaisseur de paroi de la partie centrale.

3. Dispositif de respiration artificielle trachéale (1) selon la revendication 1 ou 2, **caractérisé en ce que** les parties de tube proximale et distale (3, 4) sont espacées l'une de l'autre grâce à une fente annulaire (6) présentant une largeur de fente d faible et sont reliées entre elles grâce à l'articulation (7) recouvrant la fente (6), dans lequel le manchon (5) présente une partie de col distale (5a) non gonflable et l'articulation, de manière préférée sous la forme d'une fente annulaire (6) recouverte par au moins un élément de liaison est agencée de manière radiale à l'intérieur de ladite partie de col (5a) et au niveau d'une position axiale recouverte par la partie de col (5a) du manchon (5).

4. Dispositif de respiration artificielle trachéale (1) selon la revendication 3, **caractérisé en ce que** l'articulation (7) est formée à partir d'une partie de la paroi d'une partie de col (5a) du manchon (5).

5. Dispositif de respiration artificielle trachéale (1) selon l'une quelconque des revendications 1 à 4, **caractérisé en ce que** l'articulation (7) est élastiquement extensible et/ou compressible dans la direction axiale du tube de canule (2), dans lequel l'articulation est de manière préférée formée grâce à une partie de tuyau extensible et/ou compressible.

6. Dispositif de respiration artificielle trachéale (1) selon la revendication 5, **caractérisé en ce que** l'articulation forme au moins une partie de la paroi intérieure de la partie de manchon gonflable.

7. Dispositif de respiration artificielle trachéale (1) selon la revendication 5 ou 6, **caractérisé en ce que** la variation de la largeur de fente axiale d d'une fente annulaire entre la partie de tube proximale (3) et la partie de tube distale (4) lors d'une déformation élastique de l'articulation est comprise entre 1 mm et 10 mm, de manière préférée entre 2 mm et 4 mm.

8. Dispositif de respiration artificielle trachéale (1) selon l'une quelconque des revendications 1 à 7, **caractérisé en ce que** la partie de tube distale (4) présente grâce à l'articulation (7) un angle de flexion maximal situé dans une plage comprise entre au moins +10° et -10 °, de manière préférée une plage comprise entre +25° et -25°, par rapport à l'axe de la partie de tube proximale (3).

9. Dispositif de respiration artificielle trachéale (1) selon l'une quelconque des revendications 1 à 8, **caractérisé en ce que** l'angle d'articulation entre la partie de tube proximale (3) et la partie de tube distale (4) peut être modifié de 10° avec une force F inférieure d'au moins 70 % à celle nécessaire pour plier la partie de tube proximale (3) de 10° sans articulation.

10. Dispositif de respiration artificielle trachéale (1) selon l'une quelconque des revendications 1 à 9, dans lequel le tube de canule (2) est réalisé en matière plastique, **caractérisé en ce que** l'articulation (7) est une partie de tuyau flexible (8) qui est réalisée en une matière plastique présentant une dureté Shore inférieure à celle de la matière plastique du tube de canule (2) et qui relie la partie de tube proximale (3) à la partie de tube distale (4), dans lequel l'articulation (7) présente de manière préférée une seconde partie de tuyau flexible (8) agencée de manière concentrique par rapport à la première partie de tuyau flexible (8), dans lequel la première partie de tuyau flexible (8) est reliée de manière radiale vers l'extérieur à la partie de tube proximale (3) et à la partie de tube distale (4), et la seconde partie de tuyau flexible (8) est reliée de manière radiale vers l'intérieur à la partie de tube proximale (3) et à la partie de tube distale (4).

11. Dispositif de respiration artificielle trachéale (1) selon la revendication 10, **caractérisé en ce que** la première partie de tuyau flexible et/ou la seconde partie de tuyau flexible chevauchent les parties de tuyau flexible proximale et distale, la région de chevauchement étant de manière préférée comprise entre 2 mm et 6 mm de chaque côté de la fente.

12. Dispositif de respiration artificielle trachéale (1) selon la revendication 10 ou 11, **caractérisé en ce que** la première partie de tuyau flexible (8) et/ou la seconde partie de tuyau flexible (8) présente une épaisseur de paroi réduite par rapport à l'épaisseur de paroi moyenne de la partie de tube proximale (3) dans la région de raccordement de l'articulation (7), dans lequel l'épaisseur de paroi réduite est de manière préférée comprise entre 0,1 mm et 0,4 mm.

13. Dispositif de respiration artificielle trachéale (1) selon les revendications 5 à 10, **caractérisé en ce que** la partie de tuyau flexible (8) relie entre elles la partie de tube proximale (3) et la partie de tube distale (4) de manière ajustée.

14. Dispositif de respiration artificielle trachéale selon l'une quelconque des revendications 1 à 13, **caractérisé en ce que** l'articulation (7) présente une pluralité de nervures en matière plastique (9) qui relient la partie de tube proximale (3) à la partie de tube distale (4).

15. Dispositif de respiration artificielle trachéale (1) selon l'une quelconque des revendications 1 à 14, **caractérisé en ce que** la largeur de fente axiale d de la fente annulaire (6) est comprise entre 1 mm et 10 mm, de manière préférée entre 2 mm et 4 mm, dans lequel l'articulation (7) est de manière préférée collée, soudée et/ou moulée par injection au niveau de la partie de tube proximale (3) et/ou de la partie de tube distale (4).
